(19) Europäisches Patentamt
European Patent Office
Office européen des brevets

(11) EP 4 740 936 A1

(12) EUROPEAN PATENT APPLICATION
published in accordance with Art. 153(4) EPC

(43) Date of publication:
**13.05.2026 Bulletin 2026/20**

(21) Application number: **24854151.8**

(22) Date of filing: **07.08.2024**

(51) International Patent Classification (IPC):
***A61K 9/06*** *(2006.01)* ***A61K 31/722*** *(2006.01)*
***A61K 47/36*** *(2006.01)* ***A61L 31/14*** *(2006.01)*
***A61P 11/02*** *(2006.01)* ***C08B 37/08*** *(2006.01)*

(52) Cooperative Patent Classification (CPC):
**A61K 9/06; A61K 31/722; A61K 47/36;**
**A61L 31/14; A61P 1/04; A61P 11/02;** C08B 37/003

(86) International application number:
**PCT/JP2024/028184**

(87) International publication number:
**WO 2025/037561 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.08.2023 JP 2023132965**

(71) Applicants:
- **Kagoshima University**
  **Kagoshima-shi, Kagoshima 890-8580 (JP)**
- **iCUREX Co., Ltd.**
  **Kagoshima-shi, Kagoshima 890-0065 (JP)**

(72) Inventors:
- **TAKEI Takayuki**
  **Kagoshima-shi, Kagoshima 890-8580 (JP)**
- **YOSHIDA Masahiro**
  **Kagoshima-shi, Kagoshima 890-8580 (JP)**
- **YAMASHITA Yusuke**
  **Kagoshima-shi, Kagoshima 890-8580 (JP)**
- **HOSOYA Kei**
  **Tokyo 113-8602 (JP)**

(74) Representative: **v. Bezold & Partner**
**Patentanwälte - PartG mbB**
**Ridlerstraße 57**
**80339 München (DE)**

# (54) HYDROGEL FOR BODY CAVITY INJURY TREATMENT AND BODY CAVITY INJURY TREATMENT KIT

(57) A hydrogel for treatment of body cavity injury is derived from a chitosan derivative in which an aldonic acid is bound to an amino group of a glucosamine unit of chitosan by dehydration condensation. The hydrogel for treatment of body cavity injury has a granular shape. The hydrogel is injected into a body cavity using an injection device, and used for wound treatment of the body cavity. A kit for treatment of body cavity injury includes: an injection device; a tube to be connected to the injection device; and a hydrogel for treatment of body cavity injury, filled in the injection device and having a granular shape; wherein the hydrogel for treatment of body cavity injury is derived from a chitosan derivative in which an aldonic acid is bound to an amino group of a glucosamine unit of chitosan by dehydration condensation.

FIG. 7

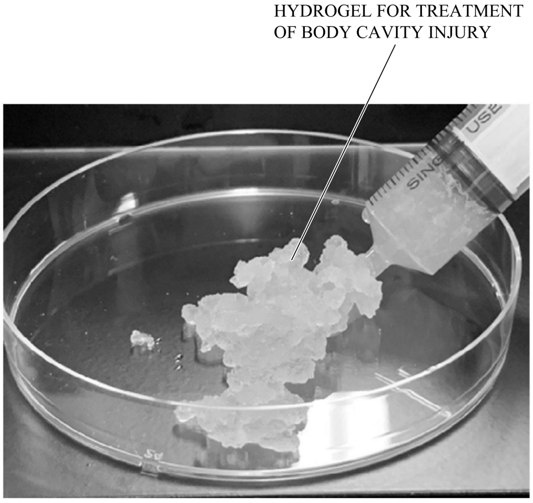




Processed by Luminess, 75001 PARIS (FR)

## Description

### Technical Field

The present disclosure relates to a hydrogel for treatment of body cavity injury, and a kit for treatment of body cavity injury.

### Background Art

A wound dressing is a medical material that covers an injury (a wound) on the body surface to keep the injury in a wet state, to thereby promote curing of the wound. A hydrogel-type wound dressing that contains a large amount of water is suitable for keeping a wound in a wet state. Chitosan has been reported to have a wound-healing effect, and a chitosan hydrogel is suitable as a wound dressing.

However, preparation of a hydrogel from chitosan involves use of a highly toxic chemical cross-linking agent, and a chitosan hydrogel obtained by such preparation is not suitable for medical purposes.

In view of this, a method of preparing a chitosan-derived hydrogel without using additives harmful to the living body has been proposed (Patent Literature 1). In Patent Literature 1, a hydrogel having no biological toxicity was prepared by freezing and thawing of an aqueous solution containing an aldonic acid-modified chitosan.

Further, Patent Literature 2 discloses use of a composition containing a soluble chitosan or derivatized chitosan for application to a wound on a body surface or body cavity.

### Citation List

### Patent Literature

Patent Literature 1: International Publication No. WO 2012/105685
Patent Literature 2: Unexamined Japanese Patent Application Publication (Translation of PCT Application) No. 2013-523827

## Summary of Invention

### Technical Problem

Patent Literature 1 is a wound dressing that covers a wound on a body surface, and there is no description at all on application of the wound dressing to a wound of a body cavity.

The composition of Patent Literature 2 is in a liquid state, and orally administered to a body cavity such as a paranasal sinus by spraying or the like. Since this composition is in a liquid state and has fluidity, the composition flows away after application to a wound site, and hence cannot be retained on the wound.

The present disclosure was made in view of the above problem, and an objective of the disclosure is to provide a hydrogel for treatment of body cavity injury, the hydrogel being also applicable to body cavity injury, and a kit for treatment of body cavity injury.

### Solution to Problem

A hydrogel for treatment of body cavity injury according to a first aspect of the present disclosure is a hydrogel for treatment of body cavity injury derived from a chitosan derivative in which an aldonic acid is bound to an amino group of a glucosamine unit of chitosan by dehydration condensation,
wherein the hydrogel for treatment of body cavity injury has a granular shape, is injected into a body cavity by an injection device, and is used for wound treatment of the body cavity.

It is preferable that the hydrogel for treatment of body cavity injury has a particle size of not more than 2 mm.

It is preferable that the aldonic acid is gluconic acid.

It is preferable that the body cavity is a nasal cavity.

A kit for treatment of body cavity injury according to a second aspect of the present disclosure includes:

an injection device;
a tube to be connected to the injection device; and

a hydrogel for treatment of body cavity injury, filled in the injection device and having a granular shape, wherein the hydrogel for treatment of body cavity injury is derived from a chitosan derivative in which an aldonic acid is bound to an amino group of a glucosamine unit of chitosan by dehydration condensation.

It is preferable that the tube has a length of 5 to 20 cm.

Advantageous Effects of Invention

According to the present disclosure, a hydrogel for treatment of body cavity injury, the hydrogel also being applicable to body cavity injury, and a kit for treatment of body cavity injury can be provided.

Brief Description of Drawings

FIG. 1 is a diagram illustrating a synthesis scheme for a chitosan derivative;
FIG. 2 is a graph illustrating the gluconic acid introduction rate in a chitosan derivative;
FIG. 3 is a perspective view of a kit for treatment of body cavity injury;
FIG. 4 is a cross-sectional view of a kit for treatment of body cavity injury;
FIG. 5 is a photograph of a hydrogel prepared by a freeze-thaw method in Examples;
FIG. 6 is a photograph of hydrogels prepared by a high-pressure steam sterilization method in Examples;
FIG. 7 is a photograph illustrating a state where a hydrogel for treatment of body cavity injury was filled in an injector and then discharged from a barrel tip;
FIG. 8 is a photograph illustrating the state of a paranasal sinus of a patient after endoscopic paranasal sinus surgery;
FIG. 9 is a photograph illustrating a state where a hydrogel for treatment of body cavity injury was injected into a paranasal sinus of a patient; and
FIG. 10 is a graph illustrating a relationship between plunger displacement and injection force.

Description of Embodiments

<Hydrogel for Treatment of Body Cavity Injury>

The hydrogel for treatment of body cavity injury according to the present embodiment is derived from a chitosan derivative in which an aldonic acid is bound to an amino group of a glucosamine unit of chitosan by dehydration condensation. The hydrogel for treatment of body cavity injury has a granular shape. The hydrogel for treatment of body cavity injury, having a granular shape, can be injected into a body cavity using an injection device and used for wound treatment of the body cavity.

By being placed on a wound surface in a body cavity, the hydrogel for treatment of body cavity injury maintains a moist environment on the wound surface and exerts a therapeutic effect on the wound surface by promoting cell proliferation. By the maintenance of the moist environment on the wound surface, formation of a scab on the wound surface can be suppressed, so that curing can be achieved earlier than by a dry therapy. In addition, since the main component is water, the hydrogel has a cooling effect, which leads to alleviation of pain and inflammation.

Since the hydrogel for treatment of body cavity injury has a granular shape, the hydrogel can be filled in a syringe of an injection device and discharged from the barrel tip of the syringe by pushing out a plunger. Therefore, the hydrogel for treatment of body cavity injury can also be injected and applied to a body cavity where application of a wound dressing is difficult.

The particle size of the hydrogel for treatment of body cavity injury is not limited as long as the hydrogel can be filled in a syringe of an injection device and discharged from the barrel tip by pushing out a plunger. The particle size is, for example, not more than 2 mm, and is preferably not more than 1 mm. In cases where the particle size of the hydrogel for treatment of body cavity injury is small, the hydrogel can be easily filled in the injection device, and can be easily discharged from the injection device.

The injection device to be used is not limited as long as the injection device has a shape that allows discharging of the hydrogel for treatment of body cavity injury. An injector generally used in the medical field can be used as the injection device. An injector of an appropriate size may be suitably selected and used depending on the particle size of the hydrogel for treatment of body cavity injury to be used and on the body cavity to which the hydrogel is to be applied. For example, a catheter tip-type injector having a large tip portion at the barrel tip may be used. An appropriate cylindrical body such as an extension member or a silicone tube may be selected for the body cavity to which the hydrogel for treatment of body cavity injury is to be applied, and the cylindrical body may be used in a state where the cylindrical body is attached to the barrel tip

of the syringe of the injector.

The hydrogel for treatment of body cavity injury can be used, for example, for wound treatment after surgical operation for sinusitis. Sinusitis is a disease in which mucosa covering a paranasal sinus is inflamed for some reason, and surgery is performed when conservative treatment with medication is not effective. In surgery for sinusitis, an endoscope is used to remove abnormal mucosa in a paranasal sinus and to open a septum of each paranasal sinus, so that paranasal sinuses are damaged. By discharging the hydrogel for treatment of body cavity injury using an injection device to place the hydrogel at the damaged site of the paranasal sinus, the damaged site can be treated.

The hydrogel for treatment of body cavity injury may be used not only for paranasal sinuses, but also for any damaged body cavity including the esophagus, the stomach (stress-induced gastric ulcer), and the large intestine.

The above-described hydrogel for treatment of body cavity injury can be produced, as described below, by granulating a hydrogel prepared by a freeze-drying method or a high-pressure steam sterilization method.

<Preparation of Hydrogel by Freeze-Drying Method, and Production of Hydrogel for Treatment of Body Cavity Injury>

In the present embodiment, the chitosan used to synthesize the chitosan derivative is a deacetylated product of chitin. As is well known, the conversion (deacetylation reaction) of chitin to chitosan does not proceed completely, and the sugar chain partially includes N-acetylglucosamine. Commercially available chitosans usually have a deacetylation rate within the range of 50 to 100%, often have a deacetylation rate of about 70 to 90%.

Unless otherwise specified, the chitosan used in relation to the present embodiment means a chitosan having a deacetylation rate of 50 to 100%, and therefore encompasses chitosans whose deacetylation rates are not 100%.

The "aldonic acid" used to synthesize the chitosan derivative used in the present embodiment is a general term of a derivative obtained by oxidizing a monosaccharide, the derivative being a carboxylic acid containing a carboxyl group derived by conversion of the formyl group at the 1-position of aldose. The aldonic acid can be represented by, for example, General Formula (I) below. In General Formula (I), n is an integer of 2 to 4.

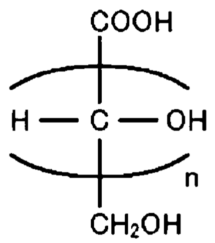

(I)

A preferred example of the aldonic acid used to synthesize the chitosan derivative used in the present embodiment is gluconic acid. Threonic acid or xylonic acid can also be preferably used. Further, any of the carboxylic acids that are known as aldonic acids may be used, and examples of such carboxylic acids include galactonic acid, mannonic acid, lyxonic acid, erythronic acid, ribonic acid, arabinonic acid, allonic acid, altronic acid, gulonic acid, idonic acid, and talonic acid.

As can be understood from the above description, the chitosan derivative used in the present embodiment can be generally represented, as shown in Formula (II) below, as a chitosan derivative containing repeat units (repeating units) each consisting of a part where aldonic acid is bound to the amino group of a glucosamine unit and a part where an acetylglucosamine unit is remaining. In Formula (II), n is an integer of 2 to 4.

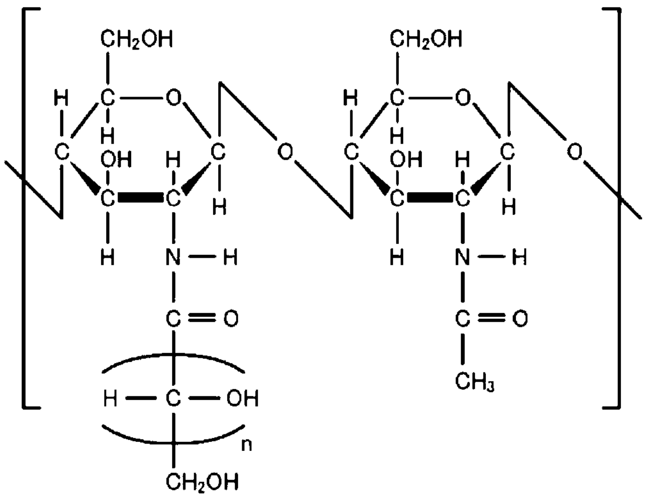

(II)

Thus, when the aldonic acid is gluconic acid, the chitosan derivative used in the present embodiment can be generally represented as a chitosan derivative containing the repeat unit shown in Formula (III) below.

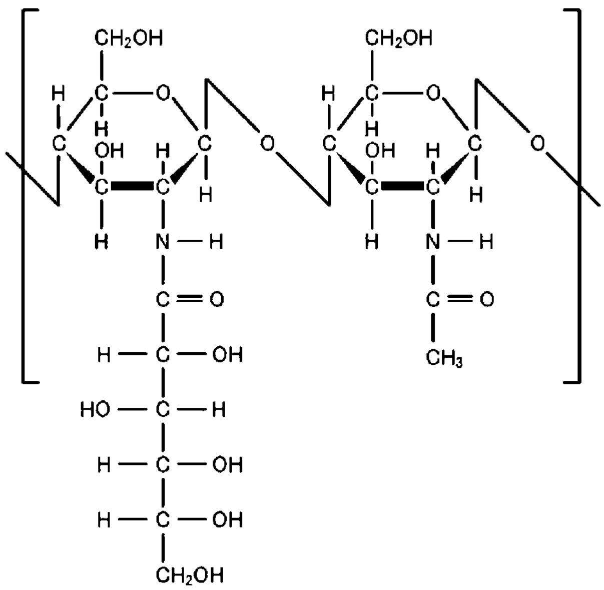

(III)

The chitosan derivative used in the present embodiment can be synthesized by subjecting aldonic acid to condensation dehydration reaction with chitosan, to bind (introduce) the aldonic acid to the amino group at the 2-position of glucosamine units of the chitosan. The aldonic acid is generally reacted as an appropriate salt (for example, Na salt). FIG. 1 illustrates a reaction scheme for a case where gluconic acid is used as the aldonic acid.

The condensation dehydration reaction can be carried out at room temperature under acidic conditions using a condensing agent (dehydration-condensation agent). Preferred examples of the condensing agent in the condensation dehydration reaction between the chitosan and the aldonic acid include, but are not limited to, 1-ethyl-3-(3-dimethylaminopropyl)carbodiimide hydrochloride (EDC), which functions to activate the carboxyl group of the aldonic acid, and N-hydroxysuccinimide (NHS), which functions to suppress side reactions.

The introduction rate of the aldonic acid such as gluconic acid into the chitosan can be adjusted by changing the amount of the aldonic acid used relative to the chitosan, and changing the amount of the condensing agent used. By increasing the relative amount of the aldonic acid used, and increasing the amount of the condensing agent used, the introduction rate can be increased. The introduction rate of the aldonic acid in the chitosan derivative used in the present embodiment is, for example, 5 to 60%.

The introduction rate of the aldonic acid such as gluconic acid (into the chitosan) is represented by Equation (A) below.

$$\text{Aldonic acid introduction rate (\%)} = [Y / (X + Y)] \times 100 \quad \text{(A)}$$

X = amount of substance (number of moles) of glucosamine units contained in the chitosan-introduced product; Y = amount of substance (number of moles) of aldonic acid-introduced glucosamine units contained in the chitosan derivative.

The introduction rate can be calculated by dissolving the chitosan derivative (aldonic acid-modified chitosan) in an appropriate concentration of aqueous hydrochloric acid solution, and then adding an equimolar concentration, with respect to the hydrochloric acid, of aqueous sodium hydroxide solution to the resulting solution while measuring the change in the electrical conductivity.

This measurement method is described below in more detail by way of FIG. 2 for the case of gluconic acid-modified chitosan, which is obtained using gluconic acid as the aldonic acid.

When 0.1 M sodium hydroxide is added to 0.1 M aqueous hydrochloric acid solution containing a gluconic acid-modified chitosan dissolved therein, $H^+$ in the solution decreases due to neutralization reaction, resulting in a decrease in the electrical conductivity of the aqueous solution (A to B in FIG. 2). Upon the completion of the neutralization reaction, protonated amino groups in the glucosamine units begin to undergo deprotonation, and the electrical conductivity does not change until the completion of the deprotonation (B to C in FIG. 2). Upon the completion of the deprotonation, $OH^-$ in the aqueous solution increases, resulting in an increase in the electrical conductivity (C to D in FIG. 2). Here, the amount of substance of the sodium hydroxide added to the aqueous solution in B to C in FIG. 2 corresponds to the amount of substance of the protonated amino groups in the glucosamine units. Taking into account the fact that the amino groups in most glucosamine units are protonated in this aqueous hydrochloric acid solution, the amount of substance of the sodium hydroxide added to the aqueous solution in B to C in FIG. 2 corresponds to the amount of substance of glucosamine units that are not modified with gluconic acid. Therefore, the gluconic acid introduction rate can be determined by calculating the amount of substance of glucosamine units in each of gluconic acid-modified chitosan and gluconic acid-unmodified chitosan by this measurement method, and comparing the calculated amounts of substance.

By pouring an aqueous solution containing the above-described chitosan derivative (aldonic acid-modified chitosan) into a mold having a predetermined shape, freezing the poured aqueous solution, and then thawing the frozen product, a hydrogel having a shape corresponding to the predetermined shape can be obtained. The freezing is performed, for example, at -20°C, and the thawing is generally performed at room temperature. However, the temperatures and the lengths of time for the freezing and the thawing are not particularly limited and may be adjusted as required.

By crushing and granulating the hydrogel prepared as described above into a predetermined particle size, a hydrogel for treatment of body cavity injury can be obtained. The crushing may be performed by any method as long as the hydrogel can be crushed into a granular shape. Examples of the method include a method in which the hydrogel is crushed using a crusher such as a mixer, and a method in which the hydrogel is crushed using a pestle.

By mixing polyvinyl alcohol (PVA) with the chitosan derivative described above and performing the same operation, a hydrogel having improved properties can also be formed. Specifically, polyvinyl alcohol is mixed with the aqueous solution containing the chitosan derivative (the aqueous solution to be poured into the casting mold), and the resulting mixture is frozen and thawed. By this, a hydrogel in which polyvinyl alcohol is mixed with the chitosan derivative is obtained.

In cases where a hydrogel in which polyvinyl alcohol is mixed with the chitosan derivative is to be obtained, the amount of polyvinyl alcohol to be blended in the hydrogel, in terms of the chitosan derivative/polyvinyl alcohol [(weight/-volume)% ratio], is preferably not less than 1/10 and less than 2/5, and is more preferably 1/5. For example, the blending ratio may be the chitosan derivative [1 (weight/volume)%] and polyvinyl alcohol [5 (weight/volume)%]. From the viewpoint of ease of gel formation, the molecular weight of the polyvinyl alcohol to be applied is preferably $6.6\times10^4$ Da (dalton) to $2.5\times10^5$ Da. For example, a polyvinyl alcohol with a molecular weight of $1.5\times10^5$ Da can be used. This is because, in cases where the molecular weight is lower than $6.6\times10^4$ Da, high strength cannot be obtained even after the gelling, while in cases where the molecular weight is higher than $2.5\times10^5$ Da, the aqueous solution has high viscosity, and hence the polyvinyl alcohol cannot be easily mixed with the chitosan derivative.

Due to contribution of the polyvinyl alcohol, the thus obtained hydrogel has properties such as extremely excellent strength, water retention capability, and enzyme degradation resistance while maintaining high biocompatibility. Therefore, for a hydrogel for treatment of body cavity injury using a hydrogel prepared by a freeze-thaw method, in cases where the hydrogel is to be used in an application requiring strength, water retention capability, and enzyme degradation resistance, polyvinyl alcohol is preferably blended in the hydrogel. By this, a decrease in the gel strength can be suppressed while the biocompatibility is maintained, and the wound can be kept in a wet state for a longer period of time.

<Preparation of Hydrogel by High-Pressure Steam Sterilization Method, and Production of Hydrogel for Treatment of Body Cavity Injury>

An aqueous solution containing a chitosan derivative synthesized in the same manner as in the freeze-thaw method described above is placed in a mold having a predetermined shape, and subjected to high-pressure steam sterilization. By this, gelling of the aqueous solution containing the chitosan derivative occurs, and at the same time, sterilization can be achieved, resulting in formation of a biocompatible hydrogel having a predetermined shape.

The high-pressure steam sterilization can be carried out using an autoclave. The temperature and the time for this treatment are not limited as long as the sterilization is possible. The treatment may be carried out at 2 atm at not less than 121°C for not less than 15 minutes as in common autoclave sterilization, or may also be carried out for 20 minutes to 2 hours.

The high-pressure steam sterilization method has an advantage that a hydrogel can be prepared in one step by subjecting the aqueous solution containing the chitosan derivative to high-pressure steam sterilization. Therefore, the hydrogel can be easily prepared also in medical institutions and the like where an autoclave is available.

By granulating the resulting hydrogel in the same manner as in the freeze-thaw method described above, a hydrogel for treatment of body cavity injury can be obtained. The other matters are the same as in <Preparation of Hydrogel by Freeze-Drying Method, and Production of Hydrogel for Treatment of Body Cavity Injury>. Description of those matters is thus omitted.

<Kit for Treatment of Body Cavity Injury>

As illustrated in FIG. 3 and FIG. 4, a kit for treatment of body cavity injury 1 comprises an injection device 10, a tube 20, and a hydrogel for treatment of body cavity injury filled in the injection device 10.

The injection device 10 comprises a cylindrical syringe 11, a plunger 12, and a cap 13. One end in the longitudinal direction of the syringe 11 is entirely open, and a flange 11a protrudes in the outer diameter direction at the end. The plunger 12, having a piston 12a that fits the inner diameter of the syringe 11, is inserted from the opening of the syringe 11. A barrel tip 11b protrudes from the other end of the syringe 11. A removable cap 13 is attached to the barrel tip 11b.

The above-described hydrogel for treatment of body cavity injury, derived from a chitosan derivative in which an aldonic acid is bound to an amino group of a glucosamine unit of chitosan by dehydration condensation, is filled in the syringe 11. When the plunger 12 is pushed, the piston 12a is brought into close contact with the inner wall of the syringe 11 while the barrel tip 11b is plugged by the cap 13. As a result, inflow of air from the outside can be suppressed, and hence drying of the hydrogel filled in the syringe 11 can be suppressed to enable long-term storage.

The tube 20 is made of a material such as a flexible silicone. The barrel tip 11b of the syringe 11 can be inserted into an end portion of the tube 20.

The injection device 10 and the tube 20 may be a commercially available injector that is commonly used in the medical field.

When the kit for treatment of body cavity injury 1 is used, the cap 13 is removed, and one end of the tube 20 is connected to the barrel tip 11b. Thereafter, the other end of the tube 20 is applied to a body cavity to be treated with the hydrogel for treatment of body cavity injury, and the plunger 12 is pushed. By this, the hydrogel for treatment of body cavity injury in the syringe 11 is discharged from the tube 20.

The length of the tube 20 is not limited. However, in cases where the tube 20 is too short, the hydrogel may not reach a filling site of the body cavity. In cases where the tube 20 is too long, an excessive force is required to push the plunger 12. In consideration of these, the length of the tube 20 is preferably 5 to 20 cm, more preferably 5 to 15 cm.

Examples

<Synthesis of Gluconic Acid-Modified Chitosan>

After dissolving 2-morpholinoethanesulfonic acid in 300 ml of distilled water at a concentration of 23.5 mM (pH 4.0), 3.0 g of chitosan (trade name, "Chitosan LL"; deacetylation rate, 80%; manufactured by Yaizu Suisankagaku Industry Co., Ltd.) was dissolved in the resulting solution, and 1 M aqueous hydrochloric acid solution was added thereto to adjust the pH to 4.0. As illustrated in Table 1, sodium gluconate (hereinafter simply referred to as GA), 1-ethyl-3-(3-dimethylaminopropyl) carbodiimide hydrochloride (hereinafter simply referred to as EDC), and N-hydroxysuccinimide (hereinafter simply referred to as NHS) were dissolved in this aqueous solution, and the resulting solution was stirred at room temperature for 24 hours, to introduce gluconic acid to the amino group at the 2-position of glucosamine units of chitosan (see FIG. 1 (reaction formula (structural formula))).

Subsequently, 1 M aqueous sodium hydroxide solution was added to the reaction solution to adjust the pH to 8.0, and then 99.5% ethanol was added thereto to precipitate gluconic acid-modified chitosan and unreacted sodium gluconate, followed by collecting the resulting precipitate by centrifugation. Thereafter, the precipitate was sealed in a dialysis membrane, and immersed in distilled water for 1 week to remove sodium gluconate. In this process, the distilled water was replaced twice a day. Subsequently, the precipitate and the aqueous solution in the dialysis membrane were collected, and 99.5% ethanol was added thereto to precipitate gluconic acid-modified chitosan, followed by collecting the resulting precipitate. Thereafter, freeze-drying was performed to obtain a dry powder of gluconic acid-modified chitosan.

The reaction results are illustrated in Table 1 below. The gluconic acid introduction rate was calculated as mentioned above by dissolving 0.2 g of the dry powder of gluconic acid-modified chitosan in 40 ml of 0.1 M aqueous

hydrochloric acid solution, and adding 0.1 M aqueous sodium hydroxide solution to the resulting aqueous solution while measuring the change in the electrical conductivity.

[Table 1]

Preparation conditions of gluconic acid-modified chitosan, and gluconic acid introduction rate

| No. | Chitosan (g) | GA (g) | EDC (g) | NHS (g) | Gluconic acid introduction rate (%) |
|---|---|---|---|---|---|
| 1 | 3.0 | 7.73 | 6.79 | 2.04 | 18.7 |

<Preparation of Hydrogel by Freeze-Thawing>

To 10 ml of distilled water, 0.1 g of dry powder of the synthesized chitosan derivative, with a gluconic acid introduction rate of 18.7%, was added, and then 0.1 M aqueous hydrochloric acid solution was added thereto, followed by completely dissolving the powder. Subsequently, 0.1 M or 1.0 M aqueous sodium hydroxide solution was added thereto to adjust the pH to 7.0, and the resulting aqueous solution was placed in a metal mold having a star-shaped cross-section and frozen at -20°C for 12 hours. Thereafter, the frozen product was thawed by being left to stand at room temperature. As a result, a gel with a star shape corresponding to the shape of the metal mold was produced as illustrated in FIG. 5. To provide a control, gelling was similarly attempted for chitosan into which gluconic acid had not been introduced. However, gelling did not occur.

<Preparation of Hydrogel Mixed with Polyvinyl Alcohol>

Dry powder of the chitosan derivative, with a gluconic acid introduction rate of 18.7%, was dissolved in physiological saline at pH 4.0 to obtain a 2 (weight/volume)% chitosan derivative solution. In addition, a polyvinyl alcohol powder (molecular weight, $1.5\times10^5$ Da; manufactured by Sigma-Aldrich) was added to another physiological saline and dissolved under heat to obtain a 10 (weight/volume)% polyvinyl alcohol solution. The chitosan derivative solution and the polyvinyl alcohol solution were mixed at 1:1 (volume ratio), and 0.1 M or 1.0 M aqueous sodium hydroxide solution was added thereto to adjust the pH of the resulting aqueous solution to 7.0. Subsequently, 1 ml of the aqueous solution obtained was placed in a cylindrical container with an inner diameter of 11 mm and frozen at -30°C for 24 hours. Thereafter, the frozen product was thawed by being left to stand at room temperature (20°C) for 2 hours. As a result, production of a hydrogel mixed with polyvinyl alcohol was confirmed.

<Synthesis of Threonic Acid-Modified Chitosan and Xylonic Acid-Modified Chitosan, and Preparation of Hydrogel>

By the same method as in the above-described synthesis of gluconic acid-modified chitosan except that threonic acid and xylonic acid were used as aldonic acids, a threonic acid-modified chitosan and a xylonic acid-modified chitosan were synthesized. The reaction results are illustrated in Tables 2 and 3, respectively below. The introduction rate of each aldonic acid was determined by dissolving the chitosan derivative (aldonic acid-modified chitosan) in an aqueous hydrochloric acid solution, and then adding an equimolar concentration, with respect to the hydrochloric acid, of aqueous sodium hydroxide solution to the resulting solution while measuring the change in the electrical conductivity.

[Table 2]

Preparation conditions of threonic acid-modified chitosan, and threonic acid introduction rate

| No. | Chitosan (g) | Threonic acid (g) | EDC (g) | NHS (g) | Threonic acid introduction rate (%) |
|---|---|---|---|---|---|
| 2 | 0.75 | 0.97 | 1.70 | 0.51 | 24.6 |

[Table 3]

Preparation conditions of xylonic acid-modified chitosan, and xylonic acid introduction rate

| No. | Chitosan (g) | Xylonic acid (g) | EDC (g) | NHS (g) | Xylonic acid introduction rate (%) |
|---|---|---|---|---|---|
| 3 | 0.75 | 1.64 | 0.85 | 0.25 | 14.9 |

The synthesized threonic acid-modified chitosan and xylonic acid-modified chitosan were frozen at -20°C for 12 hours by the same method as in <Preparation of Hydrogel by Freeze-Thawing>, and then thawed at room temperature. As

a result, gelling of the modified chitosans was confirmed.

<Preparation of Hydrogels by High-Pressure Steam Sterilization Method>

By the same method as in the above-described preparation of a hydrogel by the freeze-thaw method, dry powders of gluconic acid-modified chitosans were obtained. The reaction results are illustrated in Table 4.

[Table 4]

Preparation conditions of gluconic acid-modified chitosan, and gluconic acid introduction rate

| No. | Chitosan (g) | GA (g) | EDC (g) | NHS (g) | Gluconic acid introduction rate (%) |
|---|---|---|---|---|---|
| 4 | 10 | 2.69 | 2.37 | 0.74 | 8 |
| 5 | 10 | 5.38 | 4.73 | 1.42 | 26 |
| 6 | 5 | 10.77 | 9.46 | 2.84 | 54 |

The dry powder of each synthesized gluconic acid-modified chitosan was added to distilled water (2.0% [weight/volume]). The powder was then completely dissolved by adding 0.1 M aqueous hydrochloric acid solution thereto. Subsequently, 0.1 M or 1.0 M aqueous sodium hydroxide solution was added thereto to adjust the pH to 7.0.

The aqueous gluconic acid-modified chitosan solution was placed in a glass container having a cylindrical shape with an inner diameter of 15 mm, and then the container was sealed. The container was then placed in an autoclave, and high-pressure steam sterilization (121°C, 2 atm) was carried out. The treatment time was set to 20 minutes, 60 minutes, or 120 minutes, respectively.

As a result, all aqueous gluconic acid-modified chitosan solutions prepared with any of the treatment times showed gelling to produce hydrogels. FIG. 6 illustrates a representative result, which is a photograph of hydrogels obtained using No.6.

<Production of Hydrogels for Treatment of Body Cavity Injury>

Each hydrogel obtained above was crushed to a particle size of not more than 1 mm using a pestle and a mortar, to obtain a hydrogel for treatment of body cavity injury. Thereafter, as illustrated in FIG. 7, the hydrogel for treatment of body cavity injury was filled into an injector and discharged from the barrel tip. All hydrogels for treatment of body cavity injury were confirmed to be capable of being discharged from the injector.

<Treatment of Sinusitis>

A hydrogel for treatment of body cavity injury filled in an injector was discharged and injected into a paranasal sinus, illustrated FIG. 8, of a patient after endoscopic paranasal sinus surgery. The hydrogel for treatment of body cavity injury used was a hydrogel for treatment of body cavity injury obtained using No.1 above. FIG. 9 illustrates a state where the hydrogel for treatment of body cavity injury was injected into the paranasal sinus. Since the hydrogel for treatment of body cavity injury is in the form of a gel, the hydrogel did not drip from the paranasal sinus after the injection, and could be retained. Thereafter, favorable wound healing could be achieved. Further, the patient did not complain of a pain or an ache due to a scab.

<Accelerated Aging Test of Hydrogel for Treatment of Body Cavity Injury>

Using the hydrogel for treatment of body cavity injury obtained using No.4 above, an accelerated aging test was performed as follows.

The produced hydrogel for treatment of body cavity injury was placed in a container, sealed, and stored at 55°C until the test.

On the test day, the hydrogel for treatment of body cavity injury was taken out from the container and filled in an injection device. The filling amount of the hydrogel for treatment of body cavity injury was 2 ml. A plastic injection device was used as the injection device. The syringe had an inner diameter of 12 mm, and the barrel tip had an inner diameter of 2 mm.

The plunger was then pushed in at 900 mm/minute (15 mm/second) to perform an extrusion test in which the hydrogel for treatment of body cavity injury was discharged from the barrel tip.

The test was carried out on the day of the production of the hydrogel for treatment of body cavity injury, one month

after the production, two months after the production, three months after the production, four months after the production, five months after the production, and six months after the production. FIG. 10 illustrates the relationship between the plunger displacement [mm] and the injection force [N] required for the extrusion on each test day.

From four months after the production, the injection force tended to increase, but the injection force still remained at a level at which an operator could sufficiently extrude the hydrogel in the practical use. Therefore, the hydrogel for treatment of body cavity injury was confirmed to be practically usable even after being filled in an injection device and stored for about six months.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2023-132965, filed on August 17, 2023, the entire disclosure of which is incorporated by reference herein.

Industrial Applicability

The present disclosure is applicable to wound treatment of a body cavity, such as wound treatment of a paranasal sinus after surgical operation for sinusitis.

Reference Signs List

1 Kit for treatment of body cavity injury

10 Injection device
11 Syringe
11a Flange
11b Barrel tip
12 Plunger
12a Piston
13 Cap
20 Tube

## Claims

1. A hydrogel for treatment of body cavity injury, the hydrogel being derived from a chitosan derivative in which an aldonic acid is bound to an amino group of a glucosamine unit of chitosan by dehydration condensation,
   wherein the hydrogel for treatment of body cavity injury has a granular shape, is injected into a body cavity by an injection device, and is used for wound treatment of the body cavity.

2. The hydrogel for treatment of body cavity injury according to claim 1, having a particle size of not more than 2 mm.

3. The hydrogel for treatment of body cavity injury according to claim 1, wherein the aldonic acid is gluconic acid.

4. The hydrogel for treatment of body cavity injury according to any one of claims 1 to 3, wherein the body cavity is a nasal cavity.

5. A kit for treatment of body cavity injury, comprising:

   an injection device;
   a tube to be connected to the injection device; and
   a hydrogel for treatment of body cavity injury, filled in the injection device and having a granular shape, wherein the hydrogel for treatment of body cavity injury is derived from a chitosan derivative in which an aldonic acid is bound to an amino group of a glucosamine unit of chitosan by dehydration condensation.

**6.** The kit for treatment of body cavity injury according to claim 5, wherein the tube has a length of 5 to 20 cm.

FIG. 1

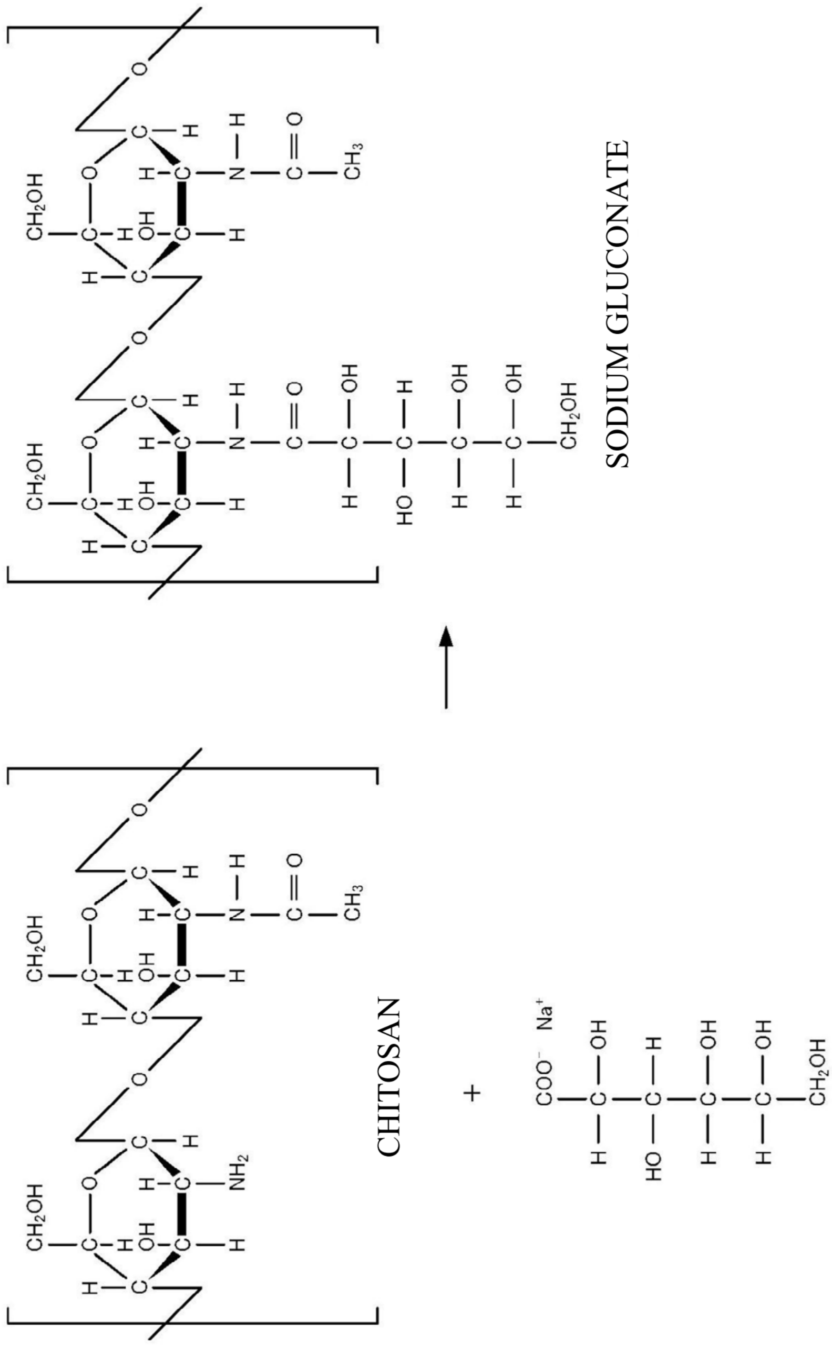

CHITOSAN

SODIUM GLUCONATE

GLUCONIC ACID-MODIFIED CHITOSAN

FIG. 2

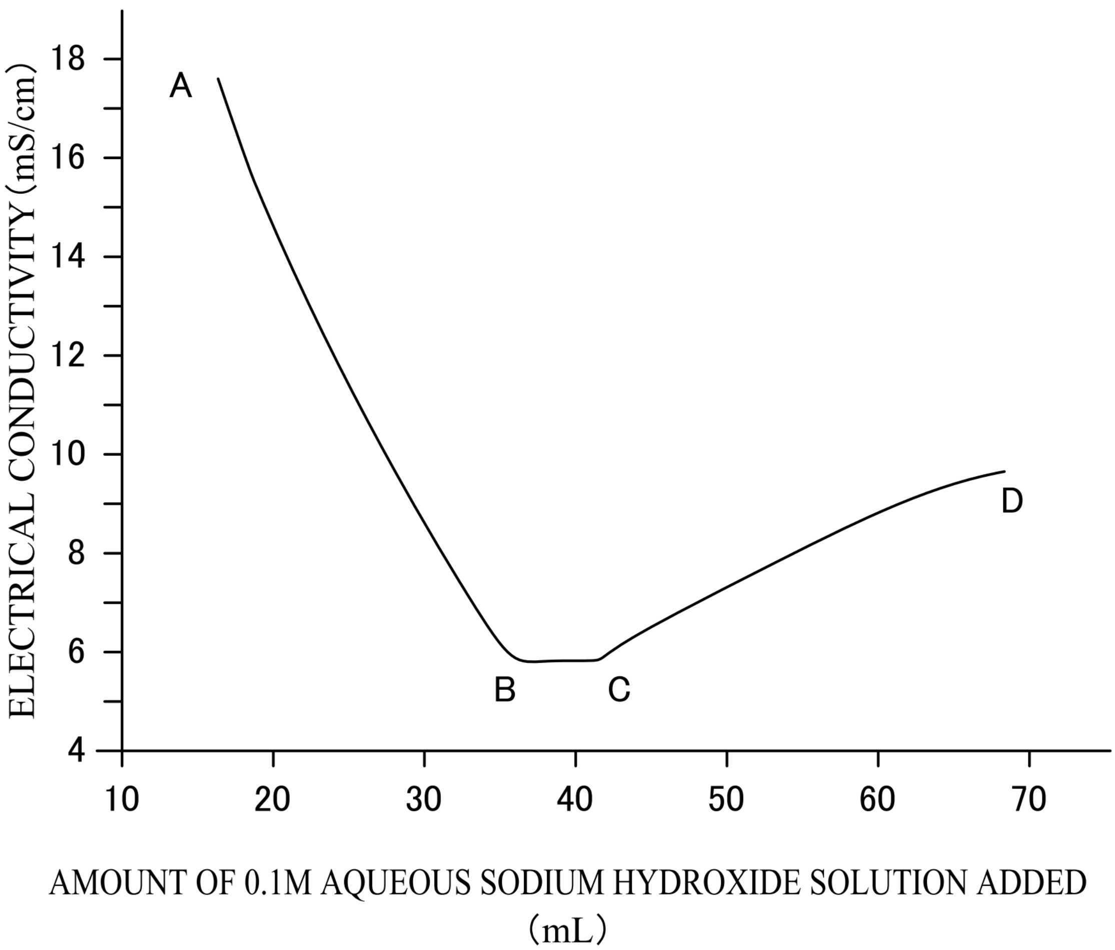
ELECTRICAL CONDUCTIVITY (mS/cm)
18
16
14
12
10
8
6
4
A
B
C
D
10
20
30
40
50
60
70
AMOUNT OF 0.1M AQUEOUS SODIUM HYDROXIDE SOLUTION ADDED
(mL)

FIG. 3

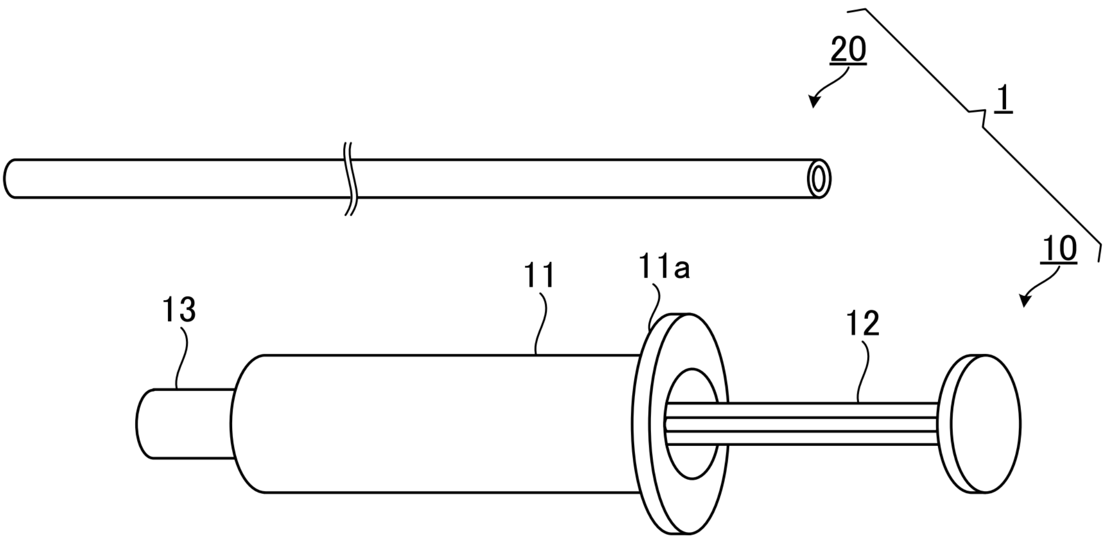

FIG. 4

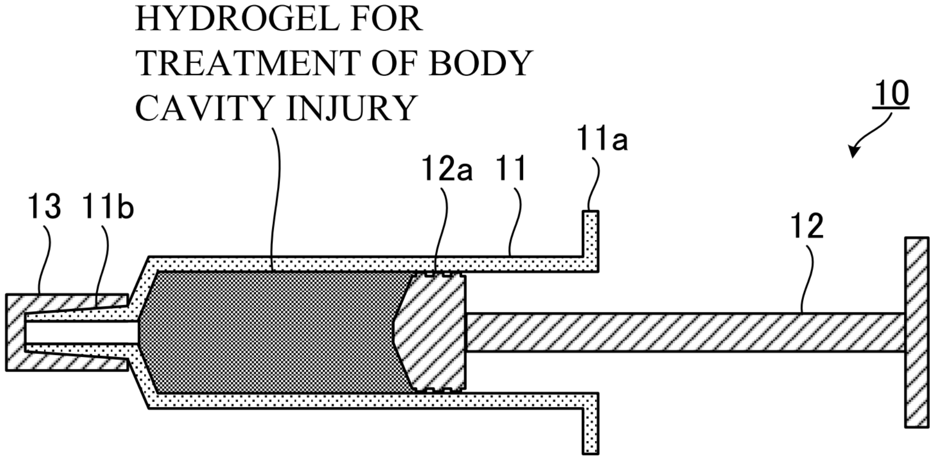
HYDROGEL FOR
TREATMENT OF BODY
CAVITY INJURY
10
11a
12a
11
13
11b
12

FIG. 5

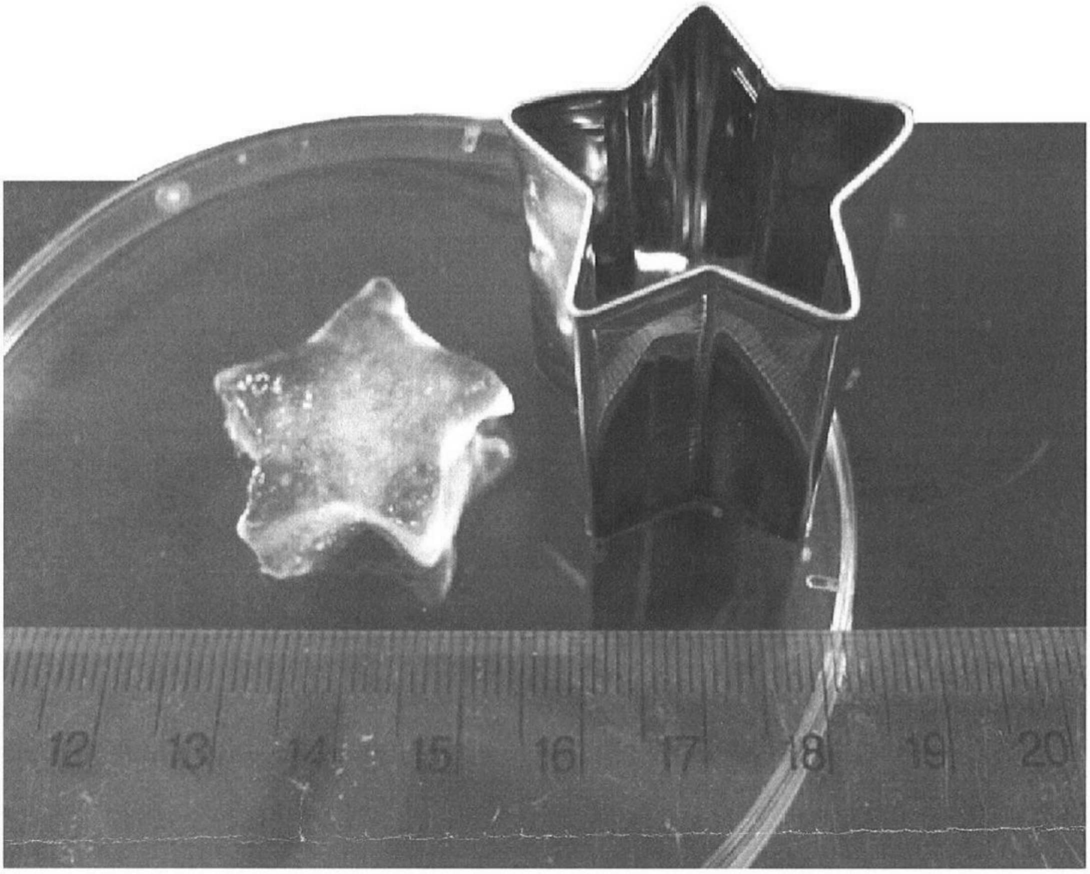

# FIG. 6

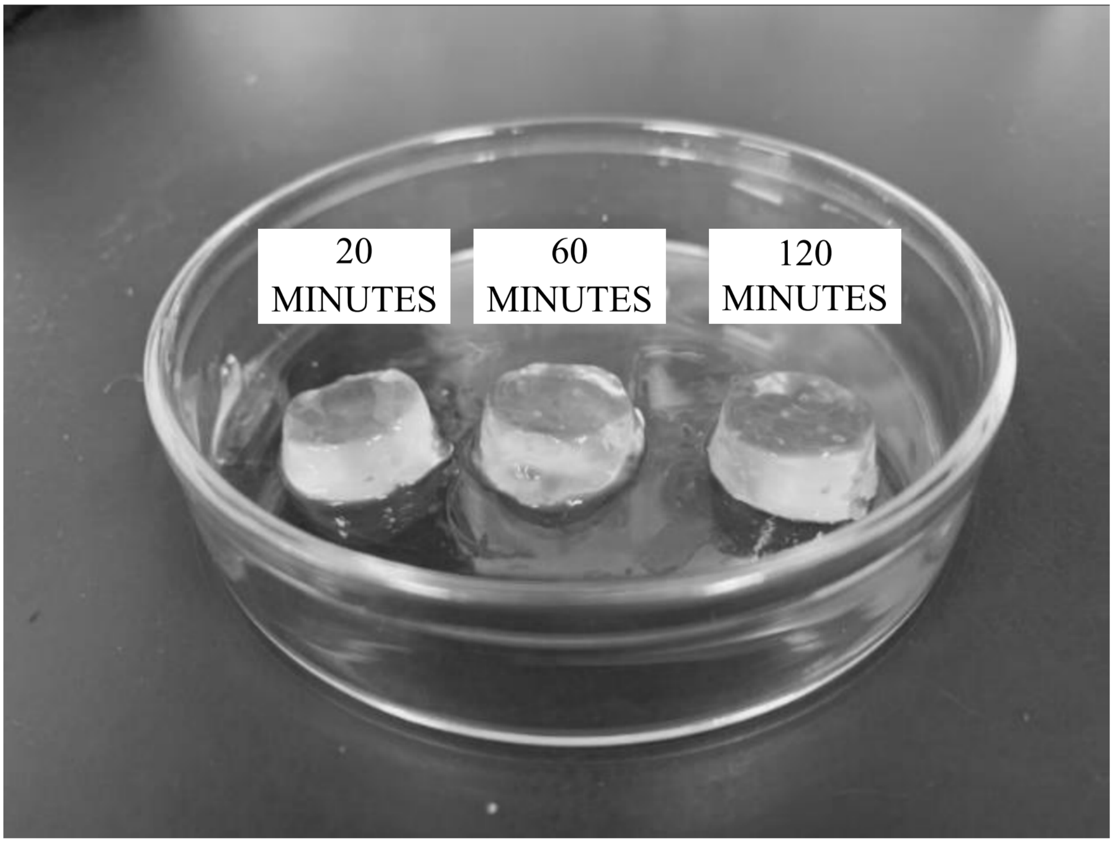

FIG. 7

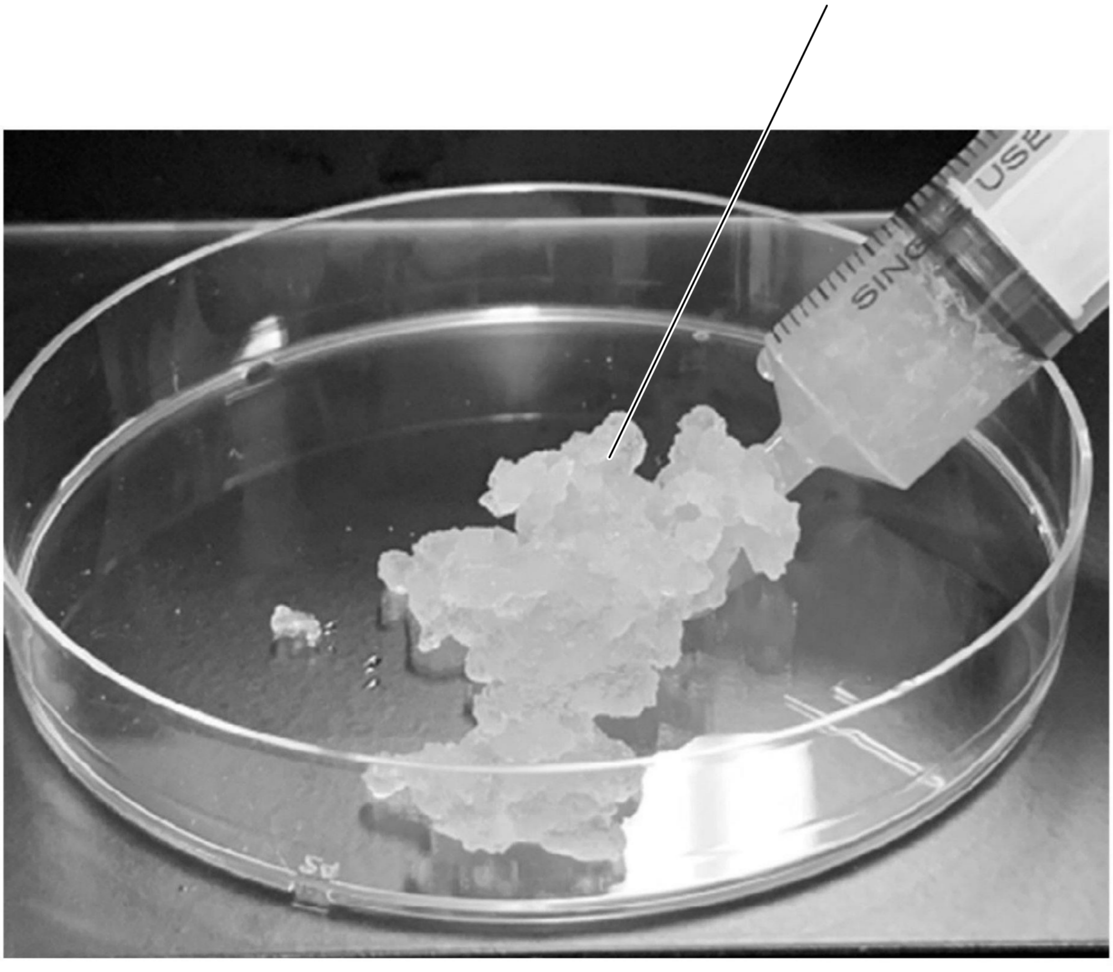

FIG. 8

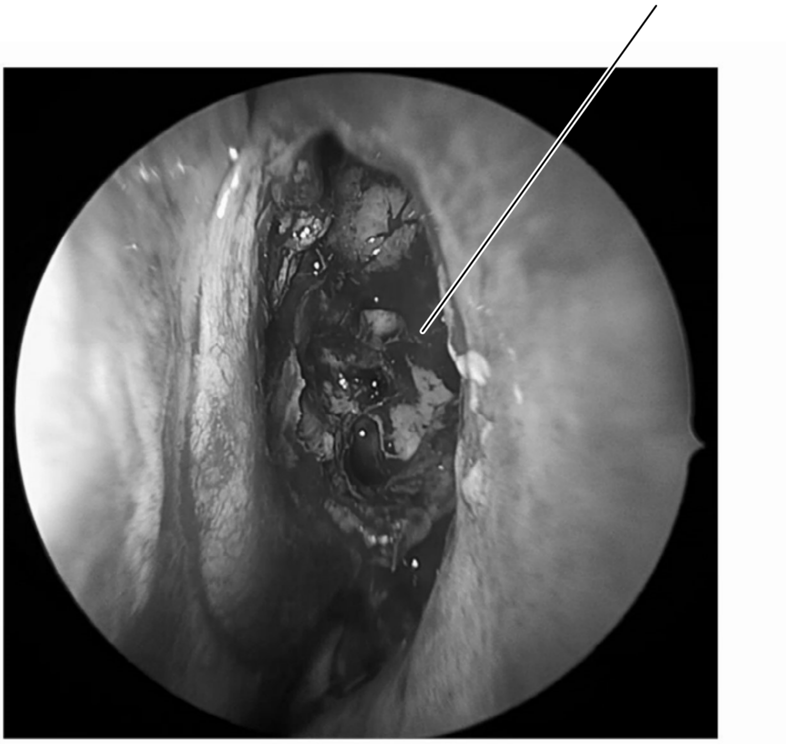

FIG. 9

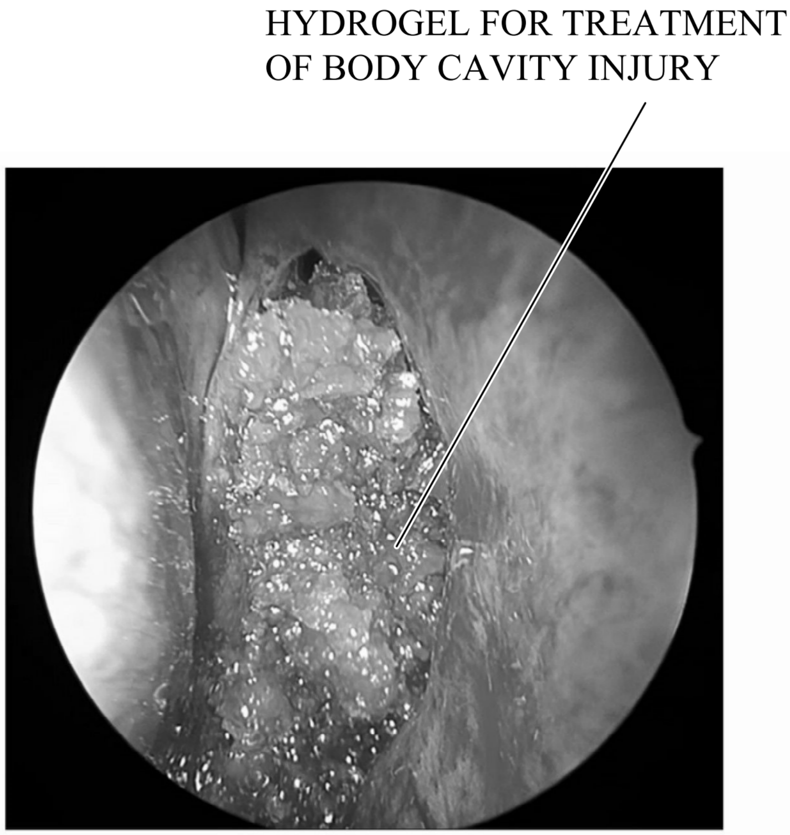

# FIG. 10

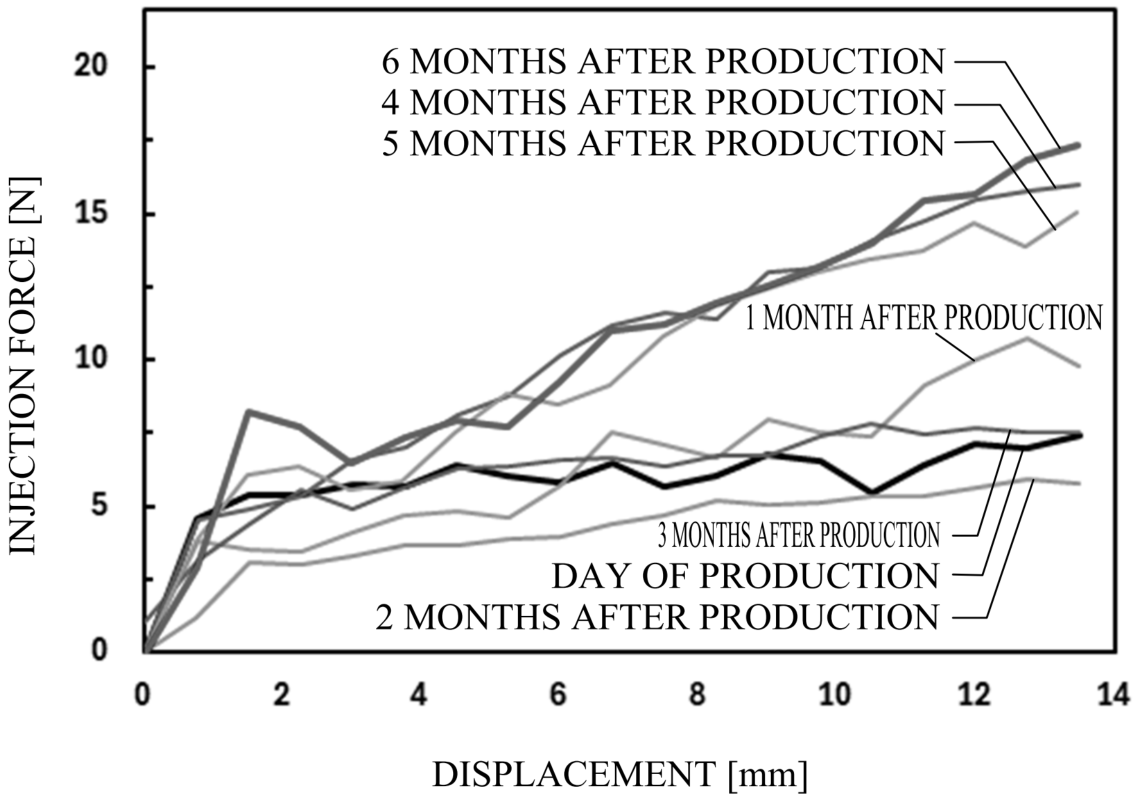

INJECTION FORCE [N]
20
15
10
5
0
0
2
4
6
8
10
12
14
DISPLACEMENT [mm]
6 MONTHS AFTER PRODUCTION
4 MONTHS AFTER PRODUCTION
5 MONTHS AFTER PRODUCTION
1 MONTH AFTER PRODUCTION
3 MONTHS AFTER PRODUCTION
DAY OF PRODUCTION
2 MONTHS AFTER PRODUCTION

**INTERNATIONAL SEARCH REPORT**

International application No.
**PCT/JP2024/028184**

**A. CLASSIFICATION OF SUBJECT MATTER**

***A61K 9/06***(2006.01)i; ***A61K 31/722***(2006.01)i; ***A61K 47/36***(2006.01)i; ***A61L 31/14***(2006.01)i; ***A61P 11/02***(2006.01)i; *C08B 37/08*(2006.01)n
FI: A61K9/06; A61P11/02; A61K47/36; A61K31/722; A61L31/14 300; C08B37/08 A

According to International Patent Classification (IPC) or to both national classification and IPC

**B. FIELDS SEARCHED**

Minimum documentation searched (classification system followed by classification symbols)

A61K9/06; A61K31/722; A61K47/36; A61L31/14; A61P11/02; C08B37/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2012/105685 A1 (KYUSHU UNIVERSITY) 09 August 2012 (2012-08-09) claims, paragraphs [0008]-[0011], [0027]-[0033], examples | 1-6 |
| Y | JP 2011-500955 A (VISCOGEL, AB) 06 January 2011 (2011-01-06) claims, paragraphs [0002], [0016], [0021], [0037], [0052]-[0053], [0065]-[0073], [0090], examples | 1-6 |
| P, A | WO 2023/167123 A1 (KAGOSHIMA UNIVERSITY) 07 September 2023 (2023-09-07) claims, paragraphs [0015]-[0016], examples | 1-6 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 October 2024** | **29 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/028184**

| Patent document cited in search report | Publication date (day/month/year) | Patent family member(s) | Publication date (day/month/year) |
|---|---|---|---|
| WO 2012/105685 A1 | 09 August 2012 | (Family: none) | |
| JP 2011-500955 A | 06 January 2011 | US 2010/0316715 A1<br>claims, paragraphs [0002], [0016], [0021], [0037], [0050]-[0051], [0065]-[0073], [0090], examples<br>WO 2009/056602 A1<br>EP 2209813 A1<br>AU 2008320877 A1<br>CA 2704162 A1<br>KR 10-2010-0083836 A<br>CN 101903408 A | |
| WO 2023/167123 A1 | 07 September 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012105685 A
- JP 2013523827 A
- JP 2023 A
- JP 132965 A